(19) Europäisches Patentamt
European Patent Office
Office européen des brevets

(11) Publication number: **0 178 913 B1**

(12) **EUROPEAN PATENT SPECIFICATION**

(45) Date of publication of patent specification: **06.03.91**

(51) Int. Cl.5: **C07C 69/28**, C07C 67/26, B01J 31/02

(21) Application number: **85307429.2**

(22) Date of filing: **15.10.85**

(54) **Preparing glycol monoesters.**

(30) Priority: **15.10.84 US 660728**

(43) Date of publication of application: **23.04.86 Bulletin 86/17**

(45) Publication of the grant of the patent: **06.03.91 Bulletin 91/10**

(84) Designated Contracting States: **BE DE FR GB IT NL**

(56) References cited:
**DE-A- 2 024 050**
**GB-A- 1 119 897**

**PATENT ABSTRACTS OF JAPAN, vol. 7, no. 248 (C-193)[1393], 4th November 1983 & JP-A-58134049**

(73) Proprietor: **EXXON RESEARCH AND ENGINEERING COMPANY**
**P.O.Box 390, 180 Park Avenue**
**Florham Park, New Jersey 07932(US)**

(72) Inventor: **Godwin, Allen David**
**Avenue de L'Orangerie 9**
**B-1410 Waterloo(BE)**

(74) Representative: **Dew, Melvyn John et al**
**Esso Chemical Ltd. Esso Chemical Research Centre P.O. Box 1**
**Abingdon Oxfordshire, OX13 6BB(GB)**

**EP 0 178 913 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates generally to the preparation of esters of carboxylic acids and more particularly to a process for preparing glycol monoesters of sterically hindered carboxylic acids in very high selectivity.

British Patent 1,119,897 relates to a process for reaction of an alkylene oxide with a sterically hindered carboxylic acid, which acid selectively favors the formation of the glycol monoester while inhibiting the formation of undesirable by-products. Catalyst disclosed for this purpose are alkali metal hydroxides, alkaline earth metal hydroxides, salts of strong basis, sodium metal, sodium alkoxides (such as sodium methylates) and, under certain conditions, mineral acids, such as sulfuric acid. Sodium hydroxide is said to be preferred.

While this method permits the efficient production of such glycol monoesters, it has not been possible heretofore to form the glycol monoesters in carboxylic acid conversions greater than about 90% without the formation of undesirably large amounts of by-product such as the di(carboxylic acid) ester of the glycol. For example, in the first example of British 1,119,897, the monoester was produced in high yield (81.7%), except that 7% of the unreacted neodecanoic acid and 11.1% diester and 2-mole ethoxylate impurities were present. Also, in the second example as the neo acid conversion approaches 100%, the concentration of the monoester rapidly decrease from 94.5% to 35%, with substantial increases in both diester and the 2-mole ethoxylated products effected.

Continued addition of ethylene oxide yielded higher ethoxylated products. The same effect, the very sudden and rapid decrease in a monoester concentration, as the conversion of the neoacid approaches 100%, is also noted in Example 3. These examples illustrate the difficulties in obtaining a high purity monoester product using the prior art process. The exact amount of ethylene oxide added to the reaction is required by the prior art patent to be carefully monitored in order to obtain monoester in high yield. Also, terminating the reaction before complete neo acid conversion is achieved is required in order to obtain monoester in an acceptable yield. This results in costly separation schemes in order to remove the unreacted neo acid from the desired monoester product.

In addition, the use of potassium or sodium hydroxides as catalyst has the disadvantage of forming water of reaction arising as a result of the neutralization of the neo acid reactant and the alkali metal hydroxide catalyst. Since water is an undesired contaminant, which can lead to by-product formations, the control of this water content is a process disadvantage.

Whilst German Patent 2024050.0 is concerned with the production of alkylene glycol esters from straight or branched chain aliphatic or cycloaliphatic residues it is not concerned with sterically hindered acids and is primarily concerned with the linear acids to which the technology of U.K. Patent 1,119,897 does not apply and suggests the use of certain amine catalysts with this sort of acid.

An improved process for preparing glycol monoesters is provided which comprises reacting an alkylene oxide with a sterically hindered carboxylic acid in the presence of an amine catalyst.

It has been surprisingly found that the use of the amine catalyst of this invention permits the selected glycol monoester to be formed in very high selectivities at high conversions of the sterically hindered acid which is charged, thereby minimizing or substantially avoiding the formation of undesirable by-products such as the glycol diesters. Unlike prior art processes, it is not necessary when using the method of this invention to avoid the presence of excess alkylene oxide or to terminate the esterification reaction prior to complete conversion of the carboxylic acid charged. It has been surprisingly found by use of the process of this invention that the di-ester by-products are not rapidly formed after consumption of the sterically hindered carboxylic acid. Therefore, the need to perform the extensive and time-consuming periodic analyses of the reaction mixture to determine the end-point of glycol monoester specificity is not necessary as is the case in use of prior art methods.

In accordance with this invention, a process is provided whereby, in the presence of an anhydrous amine catalyst, an alkylene oxide is reacted with a sterically hindered carboxylic acid, to form the glycol monoester in high selectivity and at high conversions of the acid charged, while minimizing the formation of undesirable by-products. Thus, the present invention provides a practical method for upgrading the production of glycol monoesters while utilizing readily available materials.

The sterically hindered carboxylic acid is defined as a carboxylic acid having a tertiary carbon atom in the alpha, beta, or gamma position with respect to the carboxylic group.

Acids applicable to the present process include any neo carboxylic acid, a neo carboxylic acid being defined herein as a carboxylic acid wherein the carboxyl group is attached to a tertiary carbon atom. By tertiary carbon atom is meant a carbon atom which does not have attached to it a hydrogen atom. In these neo acids the carbon atom attached to the carboxyl group viz., the "alpha carbon", will be connected to three organic radicals, each through a carbon atom. While monocarboxylic acids are most common, the

process of this invention will be equally applicable to reactions involving di- and polycarboxylic acids. (In short, therefore, the acids employed in this invention include alkanoic acids having a tertiary alpha carbon atom attached to each carboxylic acid group.) The term "alkanoic acid" is intended to include cyclic and acyclic compounds, either mono, di, or polycarboxy ccmpounds, as previously noted.

To clarify further the definition of a neo carboxylic acid, reference may be had to the following structure (I).

-5-

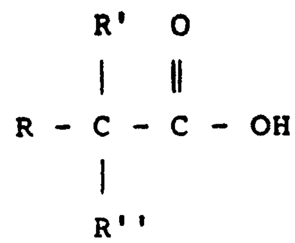

wherein each R is an organic radical.
In the most common case, R, R', and R" will independently represent an aryl, alkyl, aralkyl, or alkaryl radical of from 1 to 20 carbon atoms. The di- and polycarboxylic acids of this process will have one or more of the carboxyl groups attached to a tertiary carbon atom, e.g.,

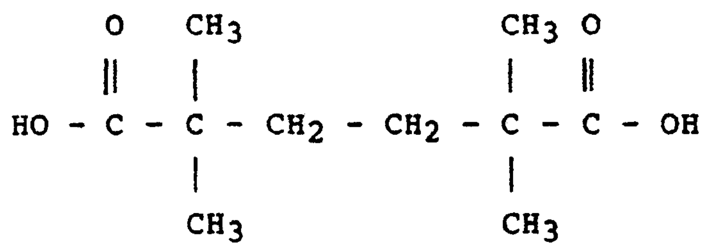

where each carboxyl group in a polycarboxylic compound is attached to a tertiary carbon atom, the compound will be fully sterically hindered. On the other hand, there are many di- and polycarboxylic acid compounds available wherein only one or, in any event, less than all, of the carboxylic groups attach to a tertiary carbon atom. In this latter case, the unhindered carboxylic group will react with an alkylene oxide in a known manner and form a random product distribution of monoesters, diesters, and higher esters. However, the sterically hindered carboxyl group will react in accordance with the present invention to yield glycol monoesters with high selectivity and yields.

In addition to the neo acid structure, other sterically hindered acids are applicable to this process. As a general rule, normal or straight chain alkanoic acids are considered to be unhindered and will react to form the random product distribution described above, whereas branching tends to create various degrees of steric hindrance. While neo acids are sterically hindered, branching on the beta and gamma carbon atoms similar to that described above for the alpha carbon atom also results in sterically hindered acids, e.g.,

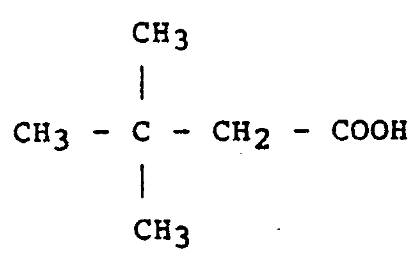

Steric hindrance is described on pages 204-217 of "Steric Effects on Organic Chemistry", edited by Melvin Newman (1956). As evidenced by this text, the branched acids, and especially those having tertiary alpha, beta or gamma carbon atoms, usually have an extremely low rate of esterification, indicating substantial steric hindrance.

Having broadly defined the carboxylic acids which may be reacted in accordance with the present process, specific examples of these hindered mono, di, and polycarboxylic acids are listed below for

purposes of illustration, it being understood that the specific list is for illustrative purposes only and not intended to define the scope of this invention. Homologues and analogues of the specific compounds listed and having a branched or tertiary alpha, beta, or gamma carbon atom are within the framework of this invention.

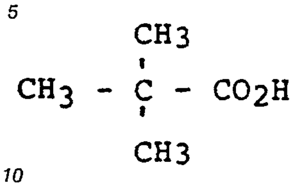, $(CH_3 - CH_2)_3 - C - CO_2H$, 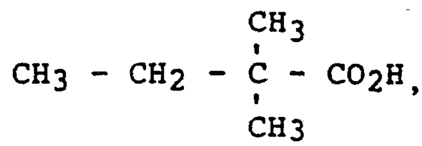,

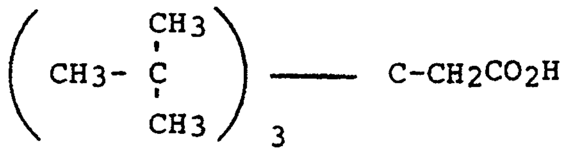

$(CH_3)_3C - CH_2CO_2H$, 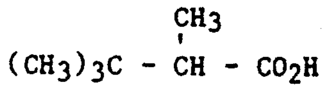

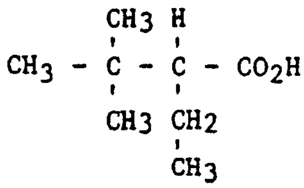, 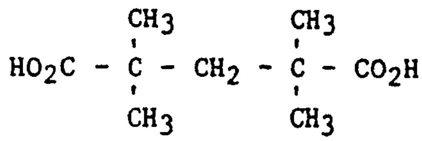,

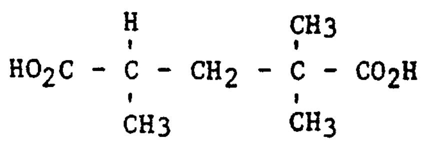,

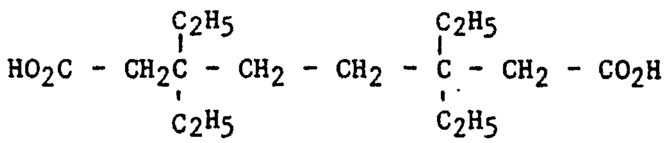,

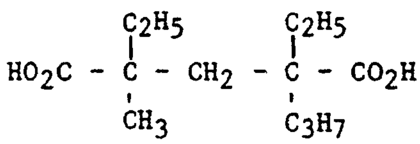,

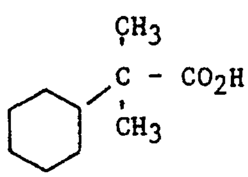, 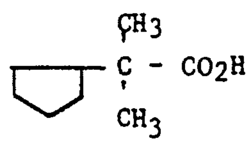,

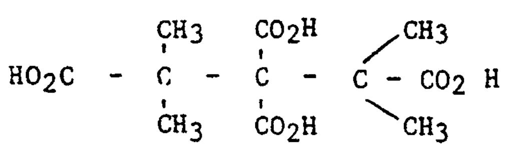,

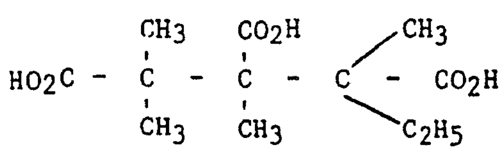, AND

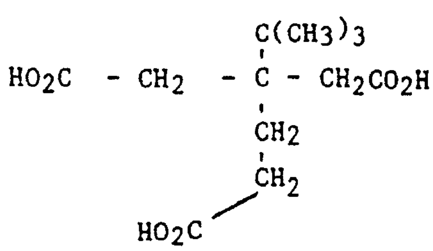

Halogen may be substituted for an alpha or beta hydrogen or R group in any of the above examples.

Preferred neo-acids comprise compounds of structure (I) wherein R' and R" are each $C_1$-$C_3$ hydrocarbyl radicals, and R is $C_1$ to $C_6$ alkyl. Some typical examples of neo-acids are: trimethylacetic acid; alpha, alpha-dimethyl cyclohexylacetic acid; alpha, alpha-dimethyl heptanoic acid; and the like. These neo-acids can also be prepared by the well known Koch process from carbon monoxide, water and type II, III, IV or V olefins as described by H. Koch in BrenntstaffChem. 36, 321 (1955). Further details on methods for making neo-acids can be found in British Patent No. 998,974 and U.S. Patent 3,349,107 all of which are incorporated herein by reference. Neo-acids are often made from branched chain olefin feedstocks which are random isomeric mixtures in regard to the position of the olefinic bond. These acids are thus random isomeric mixtures of neo-acids. These neo-acids are suitable in their isomeric forms, or any suitable mixtures thereof may be employed for the reaction to form the corresponding monoester mixtures.

The neo acids may be prepared directly from olefins, carbon monoxide, and water in a one step process. An alternative to the one step process is a two step process, whereby in the first step an olefin and carbon monoxide are reacted in the presence of an acidic catalyst, essentially in the absence of water, to form an intermediate hydrolyzable reaction product which is then hydrolyzed in the second step to liberate the desired carboxylic acid product.

The sterically hindered carboxylic acids when added to the reaction mixture are preferably free of water, e.g., the acids preferably contain<0.1wt% water.

Alkylene oxides which may be used in the present invention are the 1, 2-oxides. A preferred group within this class are the lower alkylene oxides having from 2 carbon atoms to 8 carbon atoms in the hydrocarbon chain, ethylene oxide being particularly preferred. Other representative alkylene oxides are propylene-1,2 oxide, butylene-1,2 oxide, styrene oxide, epichlorohydrin, and glycidyl ethers. It is preferred that water be kept out of the alkylene oxide since its presence will lead to the undesirable formation of polyalkylene glycol, which in turn will inhibit the process of this invention.

Water in the reaction mixture should be substantially avoided, and preferably the water content will be less than about 0.05 mol.%. The catalysts as added to the reaction mixture in the practice of this invention must be substantially anhydrous and can comprise at least one primary, secondary or tertiary amine or alkyl ammonium salts.

Aliphatic amines, alicyclic amines and aromatic amines or mixtures thereof, may be used as catalysts in this invention. These amines include primary amines of the formula $RNH_2$ wherein R is alkyl, aryl, alkaryl, aralkyl, and cycloalkyl, and secondary amines of the formula RNH(R') wherein R and R' are independently selected from the group consisting of alkyl, aryl, alkaryl, aralkyl and cycloalkyl. Also included as operable organic amines are primary and secondary amines containing fused rings and heterocyclic substituents. The primary and secondary amines may be substituted or unsubstituted, and when substituted preferably contain inert substituents such as alkyl, aryl, alkaryl, aralkyl, cycloalkyl, halogen (Cl, Br, F and I) cyano, tertiary amino, carboxyl, esters, ethers and thioether groups. Preferably, the amine reactant contains no substitution by carboxyl groups since such groups interfere with the desired ethoxylation reaction between the alkylene oxide and the selected sterically hindered carboxylic acid. The foregoing suitable hydrocarbon substituents to the R and R' groups can themselves be substituted by one or more amino groups. Exemplary of such amino substituted hydrocarbyl substituents to the R and R' groups are amino-substituted aralkyls (e.g.,

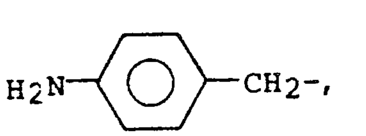 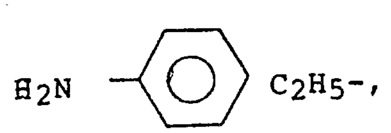

and the like), amino-substituted alkyl (e.g., $H_2NCH(CH_3)$-, $H_2NC_2H_5$- and the like) and amino-substituted alkaryls (e.g.,

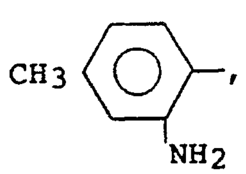 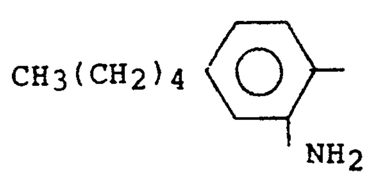

and the like).

Also included as reactants in this invention are amines of the formula $R(NH_2)m$ wherein R is as defined above and wherein m is an integer of from 2 to 5, preferably of from 2 to 3. Thus, aliphatic, alicyclic and aromatic diamines, such as ethylenediamine, hexamethylenediamine and other homologues and isomers of 2 to 10 carbon atoms are operable. The aromatic diamines include phenylenediamine, toluenediamine and naphthylenediamine. Phenylenetriamine also is operable. Polymeric amines having repeating polymer units based on any of the above amines can also be used (e.g., polymeric methylene dianiline).

Examples of R and R' substituents of the amines of each of the above formulas are alkyl radicals derived from straight and branched-chained alkanes of from 1 to 20, preferably from 1 to 12, carbon atoms (such as methyl, ethyl, isopropyl, butyl, decyl, dodecyl, iso stearyl, and the like); aryl of 6 to 18, preferably 6 to 12, carbon atoms (such as phenyl, naphthyl, anthryl and the like), alkaryl and aralkyl of 7 to 24, preferably 7 to 12, carbon atoms (such as benzyl, tolyl, p-butyl phenyl, dihexylphenyl, isostearyl phenyl, and the like) and cycloalkyl of 3 to 12 carbon atoms (such as cyclohexyl, cyclopentyl, cyclobutyl, cyclododecyl and the like). Exemplary primary amines are methylamine, ethyl amine, propylamine, ethanolamine, butylamine, isobutyamine, aniline and the like. Exemplary secondary amines are diethanolamine, butylethanol amine, diethyl amine, dimethyl amine, dibutyl amine, diphenyl amine and the like. Illustrative tertiary amines are dimethyl formamide, tri-n-propyl amine, tri-ethyl amine, dimethylethyl amine, dimethylpropyl amine, dimethylbenzyl amine, and the like. The heterocyclic amines which can be employed may contain one or more nitrogen atoms and one or more rings. Examples of amines of the character indicated which are normally liquid at room temperatures include pyridine, the alkyl-substituted pyridines, such as the picolines, the lutidines and the like, quinoline, lepidine, quinaldine and other alkyl-substituted quinolines, isoquinoline and alkyl-substituted iso-quinolines, pyrimidine, pyridazine, alkyl N-substituted heterocyclic secondary amines such as N-methyl imidazole, N-methyl piperidine, the N-methyl pipecolines, N-methyl pyrrolidine, N-methyltriazole, and the like. The alkyl substituents preferably are lower alkyl, i.e., 1 to 5 carbon atoms. Higher-melting heterocyclic amines and heterocyclic tertiary amines which are substituted by groups other than alkyl such as hydroxy, halo, alkoxy, and the groups which are nonreactive in the system are also suitably used. Examples of such catalysts are hydroxy pyridines, e.g., 2-hydroxypyridine, e.g., 2,2-bipyridine, chloropyridines such as 2-chloropyridine and like halo-substituted pyridines and quinolines, 4-methoxy-pyridine and like alkoxy pyridines and quinolines, 2-phenyl pyridine and like phenyl-substituted pyridines and quinolines, pyrazine, phenanthridine, phthalazine, quinazoline, quinoxaline, cinnoline, isoxazole, N-methyl indole, and the like. It is to be understood, however, that the foregoing named heterocyclic amines are given for illustrative purposes only.

Typical examples of suitable aromatic amine catalysts which can be used include the following primary amines such as aniline, tolyl amines, xylyl amines, naphthyl amines, anthryl amines, benzyl amine, 1- or 2-bromoethyl benzyl amine, cyclohexylamine, bis-aminoaryl-substituted alkylenes (e.g., compounds of the formula

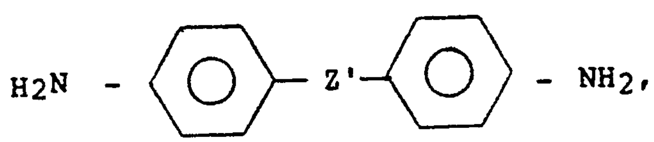

wherein Z' is alkylene of 1 to 6 carbon atoms, such as methylene, ethylene, trimethylene, tetramethylene and the like), the diamino toluenes and the like.

When the catalyst comprises an alkyl ammonium salt, the alkyl ammonium moiety of this catalyst may be represented by the formula (II)

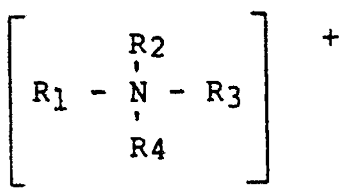

(II)

wherein $R_1$, $R_2$, $R_3$ and $R_4$ are independently selected from the group consisting of hydrogen and alkyl of 1 to 20 carbon atoms, and preferably 1 to 2 carbon atoms, provided at least two of $R_1$, $R_2$, $R_3$ and $R_4$ are alkyl. This moiety preferably contains a total of from 2 to 24 carbon atoms, and more preferably from 3 to

12 carbon atoms. The alkyl ammonium salts are preferably derived from alkyl, cycloalkyl or aryl carboxylic acids containing from 1 to 20 carbon atoms, more preferably containing from 1 to 5 carbon atoms. Exemplary of the alkyl ammonium salt catalysts of this invention, therefore, are di-, tri- and tetramethyl ammonium acetate, di-, tri-, and tetramethyl ammonium benzoate, di-, tri-, and tetramethyl ammonium n-butyrate, di-, tri-, and tetra(n-butyl) ammonium isobutyrate, di-, tri-, and tetra(isobutyl) ammonium isobutyrate, di-, tri-, and tetra propyl ammonium acetate, di-ethyl, di-methyl ammonium acetate, di-, tri-, and tetraethyl ammonium pentanoate and the like.

The alkyl ammonium may be preformed and added directly to the reaction medium, or the ammonium salt may be formed in situ. Thus, an alkyl ammonium carboxylate may be formed in situ by addition to the fluid reaction medium of the corresponding alkyl amine (in the case of di- and tri-alkyl amines), or a soluble salt containing the desired tetraalkyl ammonium moiety, and the corresponding carboxylic acid for reaction of the amine or tetraalkyl ammonium salt with the carboxylic acid. Preferably, the carboxylic acid corresponds to that acid used as the neo-acid reactant fed to the reaction mixture. If in situ formation of the promoter is desired, it is preferred to employ the desired carboxylic acid in a molar ratio of acid to amine or tetraalkyl ammonium salt of at least 1:1, and more preferably at least 2:1.

Preferred amine catalysts of this invention are dimethyl formamide, diethanol amine, butyl ethanolamine and triethanol amine.

The amine catalysts of this invention are preferably charged to the reaction mixture in an amount of from 0.1 to 5 mol.%, and more preferably from 0.5 to 2 mol.%, based on the sterically hindered neo acid charged.

The advantage of this invention lies in the ability to produce very high yields of substantially pure glycol monoesters at up to 100% neo-acid conversions, thus eliminating the necessity for costly separation procedures.

According to the present invention an alkylene oxide is reacted with a sterically hindered carboxylic acid at an elevated temperature. The optimum conditions suitable for accomplishing glycol monoester formation depend on the type of sterically hindered carboxylic acid used in the reaction mixture. A temperature range of from about 80 to 200 °C is preferably used, with temperatures of from about 140 to 185 °C being more preferred and with temperatures of from about 140 to 165 °C being especially preferred. Temperatures above 200 °C are operable; however, in practice such temperatures tend to decrease the proportion of glycol monoester in the product. Pressure is not critical but the reaction is preferablycarried out in the range of from atmospheric pressure to about 100 psig, (689.5 kPa) with a range of from atmospheric pressure to about 80 psig (552 kPa) being more preferred.

The alkylene oxide is added to the sterically hindered carboxylic acid until the reaction is complete. It is known that glycol monoester formation depends on the presence of unreacted acid. However, the use of the sterically hindered acid promotes a high degree of selectivity to the glycol monoester while inhibiting formation of higher esters which would ordinarily form if a sterically hindered acid were not used. Unreacted acid is not essential for the selective formation of the glycol monoester using the process of this invention, unlike prior art processes. Therefore, the alkylene oxide can be employed, if desired, in an excess over that stoichiometrically required to react with the sterically hindered acid. Therefore, the need for frequent and periodic analyses of the reaction mixture to establish the end point of the reaction is avoided.

It is known that reactions between alkylene oxides and carboxylic acids result in a random distribution of alkylene oxide in the products when the alkylene oxide is added in excess. The same is true in this process; however, it was discovered that by employing amine catalysts in combination with sterically hindered carboxylic acids, glycol monoester was the initial reaction product, and the rapid formation of polyalkylene oxide esters and glycol dicarboxylic acid esters which occurred in prior art processes after the consumption of the carboxylic acid, is avoided. Thus, the periodic analyses were not required. The reaction is preferably terminated when 1.03 to 1.05 molar equivalent of alkylene oxide is added.

Another advantage of this process is that undesirable inorganic salts are not present in the monoester product.

Distillation can be performed to recover the monoester reaction product formed by the method of this invention, if desired. This method of recovery is only illustrative and is not meant to limit in any way the subject of this invention. If desired, the monoester thereby obtained can also be further purified by such standard methods as: stripping at about 160 °C under vacuum, treatment with activated alumina, attapulgus clay or celite (with or without the presence of charcoal), molecular distillation and the like. Therefore, water washing, as used in the prior art, is not required.

The following Examples illustrate the invention.

EXAMPLES 1-2

Preparation of Ethylene Glycol Mononeononanoate

To a one liter stainless steel autoclave, equipped with a mechanical stirrer, heater, cooling coils, automatic temperature controller, and a tared ethylene oxide cylinder, in a series of runs is added 500 grams neononanoic acid and 2.0 g of the selected catalyst. The reactor is heated to approximately 100°c and a gaseous $N_2$ purge of the reactor is introduced to remove both oxygen and water. With continued $N_2$ purging its temperature of the neo acid/potassium hydroxide solution is gradually raised to 150°C. Upon reaching temperature, the $N_2$ purge is stopped and the reactor vents are sealed. Ethylene oxide is then slowly added to the reactor until a pressure of 60 psig (414 kPa) is obtained. The ethylene oxide flow is then stopped. As the reaction between the neo acid and the ethylene proceeds, the pressure of the reactor vessel will slowly decrease. When the pressure reaches 20 psig, ethylene oxide is again slowly charged to the reactor until a pressure of 60 psig (414 kPa) is obtained. The ethylene oxide flow is then decreased to the point where a steady reactor pressure of 60 psig (414 kPa) is obtained. The addition of ethylene oxide continues until the desired quantity of ethylene oxide has been added to the reaction. The flow of ethylene oxide is then stopped. The reaction mixture is stirred for an additional 20 minutes, until the reactor pressure decreases to 15 psig (103 kPa). Total reaction time, from the start of ethylene oxide addition is about 55 minutes. The reactor is then vented, a gaseous nitrogen purge is introduced, and the reaction product is cooled to room temperature. The crude product is distilled at 109-125°C at 15 mmHg (2 kPa) . The ethylene glycol mononeononanoate thereby obtained was analyzed by gas chromatography and found to have the following characteristics:

| | |
|---|---|
| Color, Pt/Co Scale | 30 |
| Specific gravity 20/20 | 1.0362 |
| Refractive index | 1.490 |

The results thereby obtained are set forth in Table I below:

| | |
|---|---|
| Examples 1 and 3 catalyst | = diethanolamine |
| Example 2 catalyst | = dimethyl formamide |
| Comparative Examples A, B and C catalyst | = potassium hydroxide |

TABLE I

| Example No. | Catalyst Charged | Ethylene Oxide Charged (grams) | Ethylene Oxide Charged (mole/mole neo-acid) | Product Mixture Analysis (GC)(wt%) Neo-Acid (1) | Mono-Ester (2) | Diethoxylate | Diester |
|---|---|---|---|---|---|---|---|
| 1 | DEA | 160 | 1.25 | - | 97.9 | 2.1 | - |
| 2 | DMF | 172 | 1.34 | - | 95.3 | 2.9 | 1.8 |
| 3 | DEA | 180 | 1.41 | - | 97.8 | 2.1 | <0.1 |
| Comparative Ex. A | KOH | 160 | 1.25 | - | 54.9 | 14.6 | 30.5 |
| Comparative Ex. B | KOH | 164 | 1.28 | - | 51.1 | 15.5 | 33.4 |
| Comparative Ex. C | KOH | 160 | 1.25 | - | 55.3 | 10.2 | 34.5 |

Notes:
DEA = diethanolamine; DMF = dimethylformamide; KOH = potassium hydroxide.
(1) neo-acid = neo-decanoic acid
(2) mono-ester = ethylene glycol mono-neodecanoate

## Claims

1. A process for preparing glycol monoesters by reacting an alkylene oxide with a sterically hindered carboxylic acid having a tertiary alpha, beta or gamma carbon atom in the presence of a catalyst,

characterised in that the catalyst is an amine catalyst.

2. The process according to claim 1, wherein the sterically hindered carboxylic acid comprises a neo acid containing from 1 to 20 carbon atoms.

3. The method according to claim 1 or 2, wherein the alkylene oxide comprises a 1, 2-alkylene oxide.

4. The process according to claim 3, wherein the alkylene oxide is ethylene oxide or propylene oxide.

5. The process according to any of the preceding claims, wherein the amine catalyst comprises at least one of primary, secondary and tertiary alkyl amines, aryl amines, heterocylcic amines and alkyl ammonium salts.

6. The process according to any one of the preceding claims, wherein the amine catalyst comprises at least one of ethanolamine, butylethanolamine, diethanolamine and triethanolamine.

7. The process according to any one of the preceding claims, wherein the amine catalyst is employed in an amount of from 0.1 to 5 mol%, based on the sterically hindered carboxylic acid charged to the reaction mixture.

8. The process according to any one of the preceding claims, wherein the reaction is effected at pressures of from atmospheric to 100 psig (689.6 kPa).

9. The process according to any one of the preceding claims, wherein the reaction is effected at a temperature of from 80 to 200 °C.

10. The process according to any one of the preceding claims, wherein the reaction mixture comprises less than 0.05 mol% water.

**Revendications**

1. Procédé de préparation de mono-esters de glycol par réaction d'un oxyde d'alkylène avec un acide carboxylique à encombrement stérique ayant un atome de carbone tertiaire alpha, bêta ou gamma, en présence d'un catalyseur, caractérisé en ce que le catalyseur est un catalyseur aminé.

2. Procédé suivant la revendication 1, dans lequel l'acide carboxylique à encombrement stérique comprend un néo-acide qui contient 1 à 20 atomes de carbone.

3. Procédé suivant la revendication 1 ou 2, dans lequel l'oxyde d'alkylène comprend un oxyde de 1,2-alkylène.

4. Procédé suivant la revendication 3, dans lequel l'oxyde d'alkylène est l'oxyde d'éthylène ou l'oxyde de propylène.

5. Procédé suivant l'une quelconque des revendications précédentes, dans lequel le catalyseur aminé comprend au moins un composé choisi entre des alkylamines primaires, secondaires et tertiaires, des arylamines, des amines hétérocycliques et des sels d'alkylammonium.

6. Procédé suivant l'une quelconque des revendications précédentes, dans lequel le catalyseur aminé comprend au moins une amine choisie entre l'éthanolamine, la butyléthanolamine, la diéthanolamine et la triéthanolamine.

7. Procédé suivant l'une quelconque des revendications précédentes, dans lequel le catalyseur aminé est utilisé en une quantité de 0,1 à 5 moles %, sur la base de l'acide carboxylique à encombrement stérique chargé dans le mélange réactionnel.

8. Procédé suivant l'une quelconque des revendications précédentes, dans lequel la réaction est conduite

à des pressions allant de la pression atmosphé rique à une pression manométrique de 100 lb/in$^2$ (689,6 kPa).

9. Procédé suivant l'une quelconque des revendications précédentes, dans lequel la réaction est conduite à une température de 80 à 200 °C.

10. Procédé suivant l'une quelconque des revendications précédentes, dans lequel le mélange réactionnel comprend une proportion molaire de moins de 0,05 mole % d'eau.

## Ansprüche

1. Verfahren zur Herstellung von Glykolmonoestern durch Umsetzung eines Alkylenoxids mit einer sterisch gehinderten Carbonsäure mit einem tertiären alpha-, beta- oder gamma-Kohlenstoffatom in Gegenwart eines Katalysators, dadurch gekennzeichnet, daß der Katalysator ein Aminkatalysator ist.

2. Verfahren nach Anspruch 1, bei dem die sterisch gehinderte Carbonsäure eine Neosäure umfaßt, die 1 bis 20 Kohlenstoffatome enthält.

3. Verfahren nach Anspruch 1 oder 2, bei dem das Alkylenoxid ein 1,2-Alkylenoxid umfaßt.

4. Verfahren nach Anspruch 3, bei dem das Alkylenoxid Ethylenoxid oder Propylenoxid ist.

5. Verfahren nach einem der vorangehenden Ansprüche, bei dem der Aminkatalysator mindestens eine Verbindung aus der Gruppe bestehend aus primären, sekundären und tertiären Alkylaminen, Arylaminen, heterocyclischen Aminen und Alkylammoniumsalzen umfaßt.

6. Verfahren nach einem der vorangehenden Ansprüche, bei dem der Aminkatalysator mindestens eine Verbindung aus der Gruppe bestehend aus Ethanolamin, Butylethanolamin, Diethanolamin und Triethanolamin umfaßt.

7. Verfahren nach einem der vorangehenden Ansprüche, bei dem der Aminkatalysator in einer Menge von 0,1 bis 5 Mol%, bezogen auf die der Reaktionsmischung zugesetzte sterisch gehindert Carbonsäure, verwendet wird.

8. Verfahren nach einem der vorangehenden Ansprüche, bei dem die Reaktion bei Drücken von Atmosphärendruck bis 689,6 kPa bewirkt wird.

9. Verfahren nach einem der vorangehenden Ansprüche, bei dem die Reaktion bei einer Temperatur von 80 bis 200 °C bewirkt wird.

10. Verfahren nach einem der vorangehenden Ansprüche, bei dem die Reaktionsmischung weniger als 0,05 Mol% Wasser umfaßt.